# EUROPEAN PATENT SPECIFICATION

(11) **EP 3 911 329 B1**
(45) Date of publication and mention of the grant of the patent: **14.02.2024**
(21) Application number: 20825969.7
(22) Date of filing: 16.06.2020
(51) Int. Cl.: A61K 31/519, A61K 45/06, A61P 1/16, A61P 43/00, A23L 33/10

(54) **COMPOSITION FOR PREVENTING OR TREATING METABOLIC LIVER DISEASE**
ZUSAMMENSETZUNG ZUR PRÄVENTION ODER BEHANDLUNG VON STOFFWECHSELLEBERERKRANKUNGEN
COMPOSITION POUR LA PRÉVENTION OU LE TRAITEMENT D'UNE MALADIE DU FOIE MÉTABOLIQUE

(30) Priority: 18.06.2019 KR 20190072471
(43) Date of publication of application: 24.11.2021
(73) Proprietor: Standigm Inc., Seoul 06234 (KR)
(72) Inventor: HAN, Daehee, Seoul 07002 (KR); KOO, Hee Jung, Seongnam-si Gyeonggi-do 13560 (KR); KIM, Jinhan, Seoul 03635 (KR); SONG, Sang Ok, Gyeonggi-do 13436 (KR); YUN, So Jeong, Hwaseong-si Gyeonggi-do 18442 (KR)
(74) Representative: HGF
(86) International application number: PCT/KR2020/007792
(87) International publication number: WO 2020/256382

(56) References cited:
- WO-A1-2009/018238
- WO-A1-2009/137111
- WO-A1-2018/195392
- WO-A1-2021/082683
- CN-A- 110 876 751
- US-A1- 2013 109 705
- US-A1- 2016 367 662
- US-A1- 2018 117 026
- Arturo Loaiza-Bonilla: "Dramatic response to dabrafenib and trametinib combination in a BRAF V600E-mutated cholangiocarcinoma: implementation of a molecular tumour board and next-generation sequencing for personalized medicine", ecancermedicalscience, vol. 8 , XP055767276, DOI: 10.3332/ecancer.2014.479

## Description

### Technical Field

The present invention relates to a pharmaceutical composition for preventing or treating metabolic liver disease, comprising trametinib. More specifically, the present invention relates to a composition for preventing or treating non-alcoholic fatty liver disease (NAFLD), comprising trametinib.

### Background Art

Fatty liver disease has two main types, one related to excessive alcohol consumption and the other to metabolic dysregulation. The fatty liver disease resulting from metabolic dysregulation can be metabolic liver disease, which comprises hepatic steatosis, steatohepatitis or liver fibrosis.

Non-alcoholic fatty liver disease (NAFLD) is the metabolic liver disease described above, and is a disease caused by fat accumulation in the liver that is not associated with alcohol consumption. Non-alcoholic fatty liver disease refers to the group of diseases including simple steatosis, in which only the excessive accumulation of a fat in hepatic cells is present, and the non-alcoholic steatohepatitis (NASH), which is ac-companied by necrosis, inflammation, and fibrosis of hepatic cells (Brunt EM, Non-alcoholic steatohepatitis: definition and pathology. Semin Liver Dis 21, 3-16, 2001).

Non-alcoholic steatohepatitis (NASH) occurs during the exacerbation process of non-alcohol fatty liver disease (NAFLD). First, inflammatory cytokines are secreted where the debris from destroyed hepatic cells undergoes phagocytosis by Kupffer cells and macrophages. The secreted cytokines activate hepatic stellate cells, which adjust the blood flow between hepatic sinusoid endothelial cells and hepatic cells, to synthesize and secrete connective tissue components including collagen, thereby causing the development of fibrosis. Once such process begins, it does not proceed to simple steatosis, involving fatty hepatic cells, but to non-alcoholic steatohepatitis (NASH), which is a serious lesion that causes ballooning, inflammation, or fibrosis (Ong JP et al., Obesity Surgery volume 15, pages 310-315, 2005). International patent application WO 2018/195392 describes use of trametinib to reduce the severity of abdominal adhesion due to surgical complications.

At present, there is no known composition that can act as a drug for treating metabolic liver disease, including non-alcoholic fatty liver disease, and thus there is a need to develop a therapeutic agent for treating or preventing the disease above.

Meanwhile, trametinib is an MEK inhibitor which has an anticancer action. That is, trametinib inhibits carcinogenic proteins, and in particular, is used as a drug for treating patients having BRAF V600E and V600K gene mutations. There have been no reports on any correlation between trametinib and metabolic liver disease, specifically, non-alcoholic fatty liver disease.

### Disclosure of Invention

### Technical Problem

The present inventors have made intensive efforts to develop a composition for treating or preventing metabolic liver disease, particularly, non-alcoholic fatty liver disease. As a result, they have found that trametinib, which was selected through artificial intelligence (A1) deep learning technology, alleviates steatosis, inflammation, or ballooning, and reduces the area of fibrosis due to inflammation, thereby completing the present invention.

### Solution to Problem

One object of the present disclosure not falling within the scope of the claimed invention is to provide a pharmaceutical composition for preventing or treating metabolic liver disease, comprising trametinib or a pharmaceutically acceptable salt thereof.

An object of the present invention as defined in the appended claims is to provide a pharmaceutical composition for preventing or treating non-alcoholic fatty liver disease (NAFLD), comprising trametinib or a pharmaceutically acceptable salt thereof.

Still another object of the present disclosure not falling within the scope of the claimed invention is to provide a method for treating metabolic liver disease, comprising administering the pharmaceutical composition to a subject.

### Advantageous Effects of Invention

The composition of the present invention, which comprises trametinib, reduces steatosis, inflammation, or ballooning in liver tissue where non-alcoholic fatty liver has been induced; reduces the level of fibrosis of inflammation induced by non-alcoholic fatty liver; and reduces the weight of a liver that has increased due to non-alcoholic fatty liver disease. Accordingly, the composition of the present invention can be effectively used for the prevention or treatment of metabolic liver disease, including non-alcoholic fatty liver disease.

### Brief Description of Drawings

FIG. 1 shows liver fragments stained with hematoxylin and eosin (H&E).
FIG. 2 is a figure quantifying steatosis scores (V: vehicle, T telmisartan, TR: trametinib).
FIG. 3 is a figure quantifying inflammation scores (V: vehicle, T: telmisartan. TR: trametinib).
FIG. 4 is a figure quantifying ballooning scores (V: vehicle, T: telmisartan, TR: trametinib).
FIG. 5 is a figure quantifying NAFLD activity scores (NAS). P values were measured according to the Bonferroni Multiple Comparison Test (V: vehicle, T: telmisartan, TR trametinib).
FIG. 6 shows liver fragments stained with sirius red.
FIG. 7 is a figure quantifying a percentage of a fibrosis area due to inflammation and a sirius red-positive area *P* values were measured according to the Bonferroni Multiple Comparison Test (V: vehicle, T: telmisartan, TR: trametinib).
FIG. 8 shows the body weight, liver weight, and liver-to-body weight ratio of a mouse from each group. *P* values were measured according to the Bonferroni Multiple Comparison Test (V: vehicle, T: telmisartan, TR: trametinib).
FIG. 9 shows liver fragments stained with H&E.
FIG. 10 is a figure quantifying steatosis scores (V: vehicle, T: telmisartan, TR: trametinib).
FIG. 11 is a figure quantifying inflammation scores (V: vehicle, T: telmisartan, TR: trametinib).
FIG. 12 is a figure quantifying ballooning scores (V: vehicle, T: telmisartan, TR: trametinib).
FIG. 13 is a figure quantifying NAFLD activity scores (V: vehicle, T: telmisartan, TR: trametinib).
FIG. 14 shows liver fragments stained with sirius red.
FIG. 15 is a figure quantifying a percentage of a fibrosis area due to inflammation and a sirius red-positive area *P* values were measured according to the Bonferroni Multiple Comparison Test (V: vehicle, T: telmisartan, TR: trametinib).
FIG. 16 shows the body weight, liver weight, and the liver to body weight ratio of a mouse from each group. *P* values were measured according to the Bonferroni Multiple Comparison Test (V: vehicle, T: telmisartan, TR: trametinib).

### Best Mode for Carrying out the Invention

Hereinbelow, the present invention will be explained in detail.

An aspect of the present disclosure not falling within the scope of the claimed invention for resolving the technical problem above provides a pharmaceutical composition for preventing or treating metabolic liver disease, comprising trametinib or a pharmaceutically acceptable salt thereof.

As used herein, the term "trametinib" refers to the compound N-(3-{3-cyclopropyl-5-[(2-fluoro-4-iodophenyl)amino]-6,8-dimethyl-2,4,7-trioxo-3,4,6,7 -tetrahydropyrido[4,3-d]pyrimidin-1(2H)-yl}phenyl)acetamide, which is an MEK inhibitor. Trametinib inhibits carcinogenic proteins, and in particular, is used as a drug for treating patients having BRAFV600E and V600K gene mutations.

In the present invention, it was found that the trametinib has an effect of alleviating steatosis, inflammation, or ballooning in liver tissue where non-alcoholic fatty liver has been induced, and an effect of reducing fibrosis due to inflammation induced by the disease above, and thus, the present invention is significant in discovering for the first time that the trametinib can be used in treating metabolic liver disease.

The trametinib may be a chemical compound represented by Chemical Formula I below.

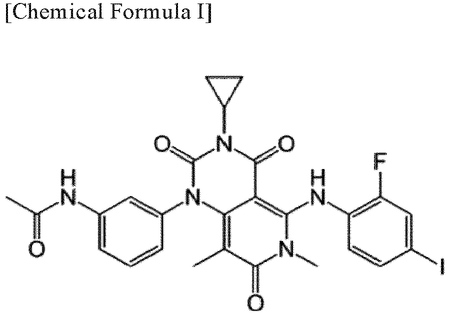

The trametinib may be commercially available through a synthetic method known in the art, but is not limited thereto.

As used herein, the term "pharmaceutically acceptable salt" refers to any organic or inorganic addition salt of the compound above at a concentration having relatively nontoxic and harmless effective actions in a subject, where side effects attributed to the salt do not deteriorate the beneficial efficacy of the compound. In addition the compound may be used alone or in combination with another pharmaceutically active compound or in an aggregate.

The pharmaceutically acceptable salt of trametinib (*i*.*e*., the compound of the present invention) refers to a salt prepared according a conventional method known in the art, and such preparation method is known to one of ordinary skill in the art. Specifically, the pharmaceutically acceptable salt may include salts which are induced from the following inorganic acids, organic acids, and bases, which are pharmacologically or physiologically acceptable, but the pharmaceutically acceptable salt is not limited thereto.

Acid addition salts may be prepared using a conventional method such as dissolving a compound in an excess aqueous acid solution and precipitating the salt using a water-miscible organic solvent such as methanol, ethanol, acetone, or acetonitrile. An equimolar compound and an acid or alcohol in water (*e.g*., glycol monomethyl ether) are heated, and then the mixture may be dried by evaporation, or the precipitated salt may be suction filtered. In particular, the free acid may include an inorganic acid or an organic acid. The inorganic acid may include hydrochloric acid, phosphoric acid, sulfuric acid, nitric acid, tartaric acid, *etc.* The organic acid may include methanesulfonic acid, *p*-toluenesulfonic acid, acetic acid, trifluoroacetic acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, hydroiodic acid, *etc.* However, the inorganic acid and organic acid are not limited thereto.

In addition, a pharmaceutically acceptable metal salt may be prepared using a base. An alkali metal salt or alkaline earth metal salt is obtained by, for example, dissolving a compound in an excess alkali metal hydroxide solution or alkaline earth metal hydroxide solution, filtering the insoluble compound salt, and evaporating and drying the filtrate. In particular, preparation of a sodium, potassium, or calcium salt as the metal salt is pharmaceutically suitable, but the metal salt is not limited thereto. Further, a corresponding silver salt may be obtained by reacting the alkali metal or alkaline earth metal salt with a suitable silver salt (*e*.*g*., silver nitrate).

In the present invention, not only the trametinib or the pharmaceutically acceptable salt thereof, but also all solvates or hydrates which can be prepared from the trametinib or the pharmaceutically acceptable salt thereof and which exhibit identical efficacy may be included in the scope of the present invention.

The pharmaceutical composition of the present invention, which comprises trametinib or a pharmaceutically acceptable salt thereof, may further include a suitable carrier, excipient, or diluent which is conventionally used in preparation of a pharmaceutical composition. The carrier may also include a non-naturally occurring carrier, but is not limited thereto.

Carriers, excipients, or diluents which can be used in the present invention may include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methylhy-droxybenzoate, propylhydroxybenzoate, talc, magnesium stearate, or mineral oil, *etc.*

The pharmaceutical composition according to the present invention may be used by being formulated into oral formulations such as powders, granules, tablets, capsules, suspensions, emulsions, syrups, aerosol, *etc.,* external solutions, suppositories, and sterile injectable solution, according to a conventional method for each thereof.

For the preparation of these formulations, the pharmaceutical composition may be formulated in combination with a commonly-used diluent or excipient such as a filler, extender, binder, humectant, disintegrating agent, surfactant, *etc.* Solid formulations for oral administration may include tablets, pills, powders, granules, capsules, *etc.,* and these solid formulations may be prepared by mixing with at least one excipient, *e*.*g*., starch, calcium carbonate, sucrose or lactose, gelatin, *etc.*

In addition to a simple excipient, a lubricant such as magnesium stearate, talc, *etc.* may be used. Liquid formulations for oral administration may include suspensions, oral solutions, emulsions, syrups, *etc.,* and, in addition to a simple diluent such as water or liquid paraffin, various excipients such as humectants, sweeteners, flavoring agents, preservatives, *etc.* may be used in the liquid formulations.

As used herein, the term "metabolic liver disease" refers to a disease caused by metabolic dysregulation in the liver that is not associated with alcohol consumption. In the present invention, the metabolic liver disease is non-alcoholic fatty liver disease (NAFLD),

An aspect of the present invention provides a pharmaceutical composition for preventing or treating non-alcoholic fatty liver disease, comprising trametinib or a pharmaceutically acceptable salt thereof.

As used herein, the term "non-alcoholic fatty liver disease (NAFLD)" refers to a disease caused by fat accumulation in the liver which is not associated with alcohol consumption. The non-alcoholic fatty liver disease may include liver triglyceride accumulation, simple steatosis, non-alcoholic fatty liver (NAFL), non-alcoholic steatohepatitis (NASH), and NAFLD-related liver fibrosis or hepatocirrhosis, which is caused by advancement of the diseases above. The non-alcoholic fatty liver disease may progress into non-alcoholic steatohepatitis or non-alcoholic steatohepatitis accompanied by fibrosis.

As used herein, the term "non-alcoholic steatohepatitis (NASH)" refers to a disease which occurs during the exacerbation process of non-alcoholic fatty liver disease (NAFLD), and in which triglyceride accumulates in the liver and the increase of Kupffer cells and the activation of phagocytes proceed in the fatty state. Subsequently, the oxidation of hepatocellular mitochondria occurs, causing inflammation and fibrosis. The main symptoms of the disease may include steatosis, inflammation, or ballooning in liver tissue, and may be accompanied by fibrosis of the liver tissue.

The "steatosis" refers to a phenomenon in which lipids accumulate due to abnormalities in lipid metabolism. The "inflammation" refers to the degree of lobular inflammation in the liver. The "ballooning" refers to ballooning of hepatic cells or degeneration caused by swelling of hepatic cells. The "fibrosis" refers to a phenomenon in which a part of the tissue is hardened.

The pharmaceutical composition of the present invention, which comprises trametinib, may reduce steatosis, inflammation, or ballooning, which are the symptoms of non-alcoholic fatty liver disease among metabolic liver diseases, or may reduce the fibrosis due to inflammation, but is not limited thereto.

The three types of foci of the steatosis, inflammation, and ballooning can be comprehensively analyzed and quantified, and this is reflected in NAFLD activity score (NAS).

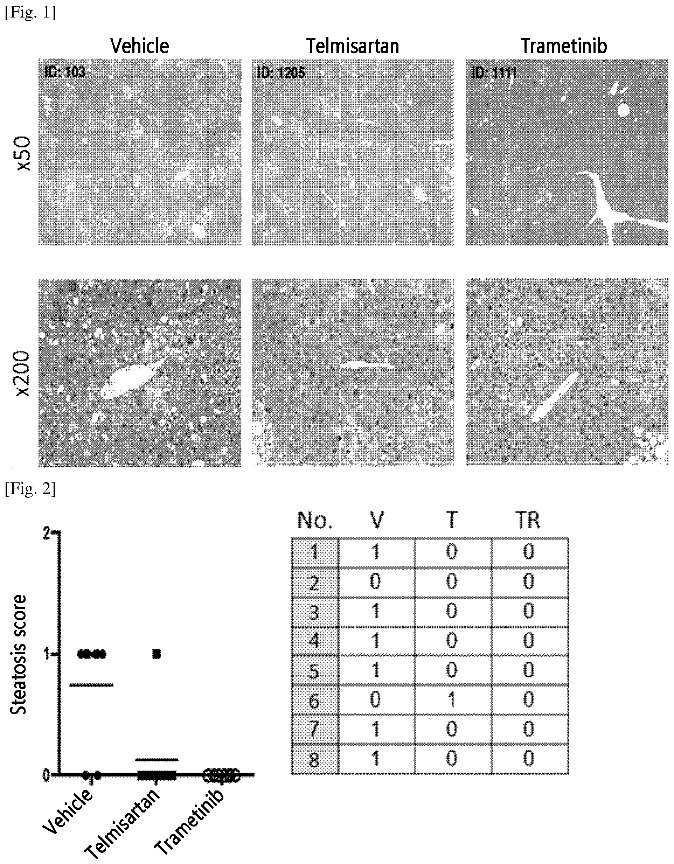

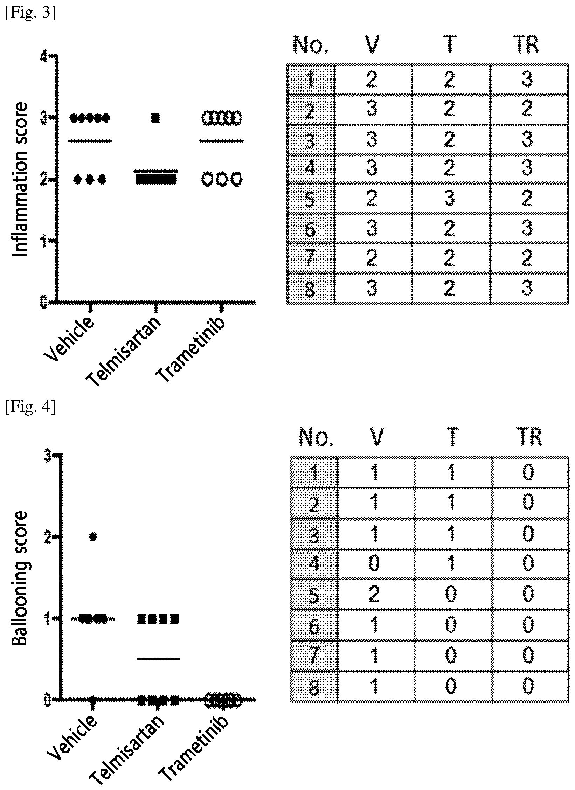

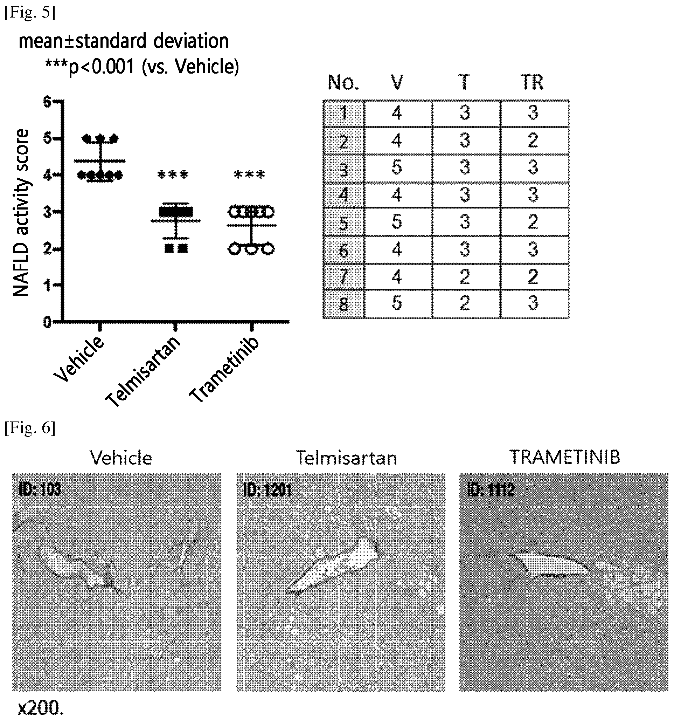

In a specific embodiment of the present invention, it was found that trametinib reduces steatosis, inflammation, or ballooning (FIGS. 2 to 5, and 10 to 13). Specifically, it was confirmed that when the trametinib of the present invention was intraperitoneally administered at a dose of 2 mg/kg, the steatosis score reduced to an average of 0 as compared to that of the vehicle administration group (an average of 0.8), which is the negative control group (FIG. 2). In particular, it was shown that when the composition of the present invention was administered, there were eight subjects with a ballooning score of 0 (*i*.*e*., evaluated as not having ballooned cells) (FIG. 4). It was confirmed that since the numerical value above corresponds to 100% of the total number of subjects, the composition of the present invention exhibits a significant effect in reducing ballooning. Accordingly, it was shown that the NAFLD activity score, which reflects ballooning, was also reduced as compared to that of the vehicle administration group (FIG. 5).

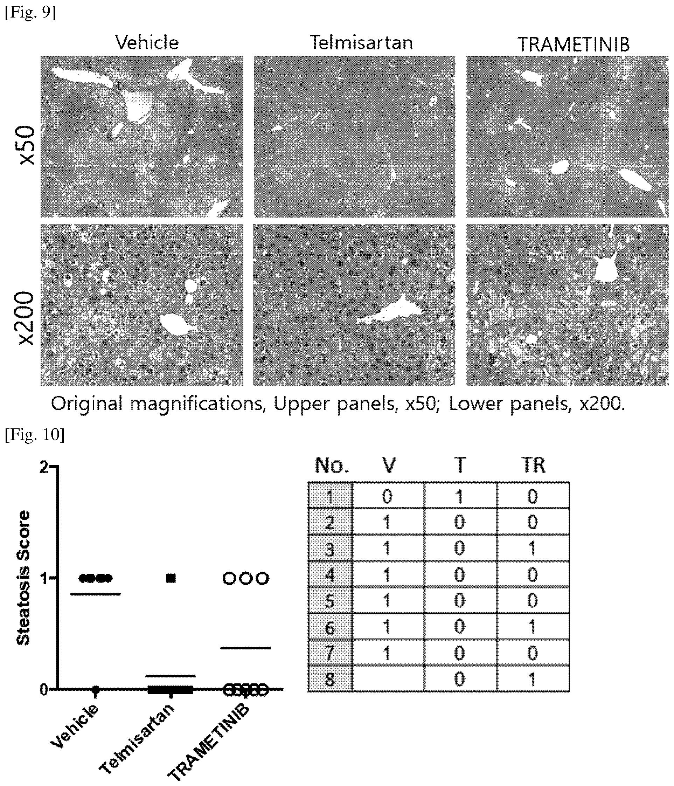

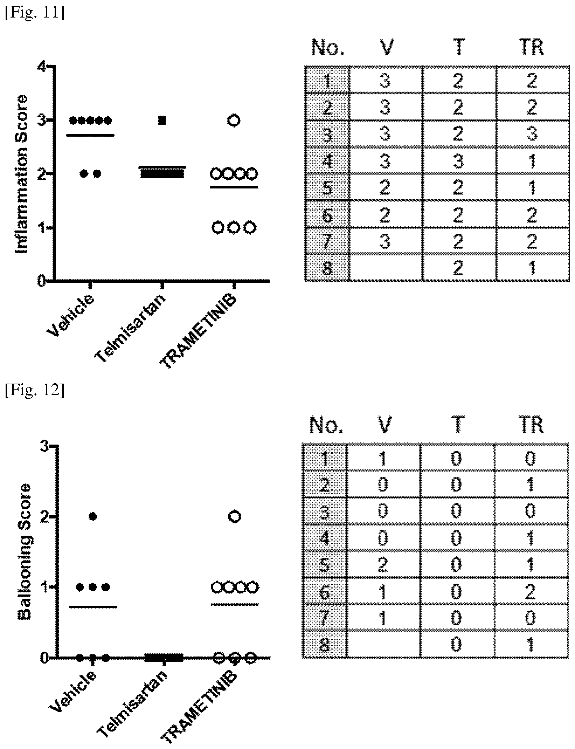

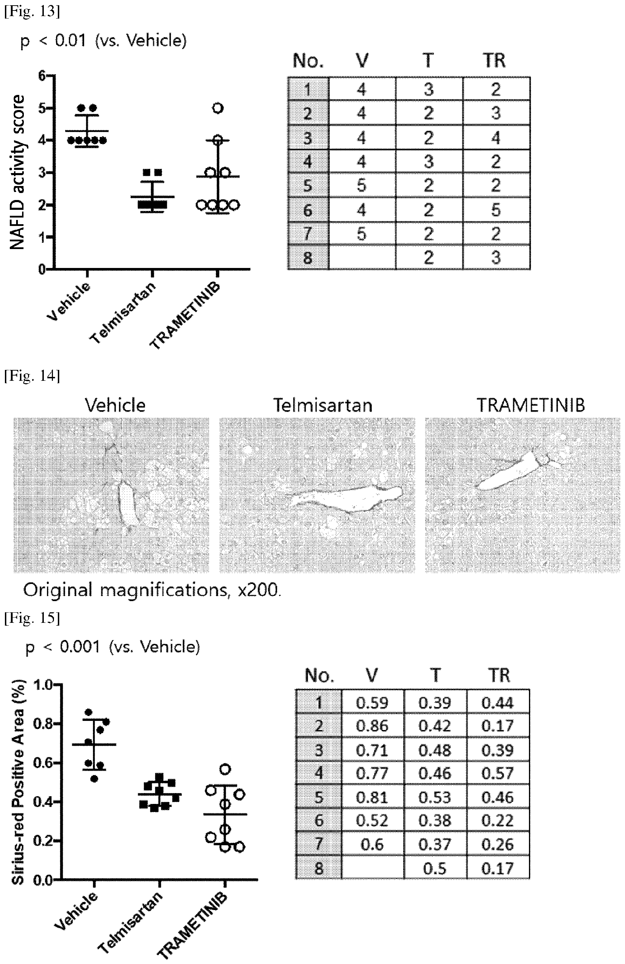

In addition, it was confirmed that when the trametinib of the present invention was orally administered at a dose of 0.2 mg/kg (in a volume of 10 mL/kg), the steatosis score was reduced to an average of 0.4 as compared to that of the vehicle administration group (an average of 0.9), which is the negative control group (FIG. 10); the inflammation score was also reduced to an average of 1.8 as compared to that of the vehicle administration group (an average of 2.7) (FIG. 11). Accordingly, it was found that the NAFLD activity score, which reflects steatosis and inflammation, was also reduced as compared to that of the vehicle administration group (FIG. 13).

That is, the trametinib of the present invention exhibited an effect of reducing the NAFLD activity score as compared to that of the vehicle administration group.

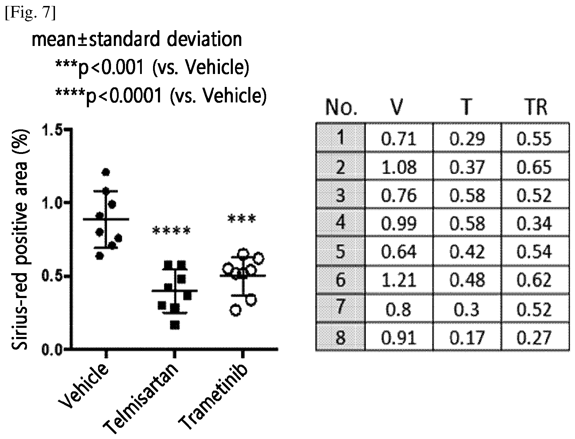

In another specific embodiment of the present invention, it was confirmed that trametinib reduced the fibrosis of inflammation induced by non-alcoholic steatohepatitis (FIGS. 6, 7, 14, and 15). Specifically, the fibrosis area score of the vehicle administration group was 0.9 on average, while that of the telmisartan administration group *(i.e.,* positive control group) was 0.4 on average, and that of the trametinib administration group was 0.5 on average, similar to that of the telmisartan administration group (FIG. 7). In addition, when the trametinib of the present invention was orally administered at a dose of 0.2 mg/kg (in a volume of 10 mL/kg), the fibrosis area score was reduced to 0.3 as compared to that of the vehicle administration group (0.7 on average), and this score was superior to that of the telmisartan administration group (0.4 on average) (FIG. 15).

That is, with regard to the fibrosis due to inflammation, the trametinib of the present invention showed an effect of reducing the fibrosis area to a level superior or similar to that of the positive control group (*i.e*., telmisartan).

Based on the results above, it was found that the pharmaceutical composition of the present invention, which comprises trametinib, has an effect of preventing or treating metabolic liver disease, specifically, non-alcoholic fatty liver disease, and more specifically, non-alcoholic steatohepatitis.

As used herein, the term "prevention" refers to any activity that inhibits or delays the occurrence, spread, or recurrence of a subject disease by administration of the pharmaceutical composition, and the term "treatment" refers to any activity associated with the amelioration or advantageous changes in symptoms of a subject disease by administration of the pharmaceutical composition.

In a specific embodiment of the present invention, the treatment may achieve reduction or alleviation of signs of metabolic liver disease, specifically, non-alcoholic fatty liver disease and/or non-alcoholic steatohepatitis, reduction of the severity of the disease, delay or slowing of the disease, palliation or stabilization of the disease condition, or other advantageous results, but the treatment is not limited thereto.

Still another aspect of the present invention provides a method for preventing or treating metabolic liver disease including non-alcoholic fatty liver disease, comprising administrating the pharmaceutical composition to a subject.

The terms "pharmaceutical composition", "non-alcoholic fatty liver disease", "metabolic liver disease", "prevention", and "treatment" are as described above.

As used herein, the term "subject" refers to all animals including humans that have or may develop a subject disease, and by administering the pharmaceutical composition of the present invention to a subject suspected of having or suffering from metabolic liver disease, the disease may be effectively treated or prevented. The pharmaceutical composition of the present invention may be applied to any subject without particular limitation as long as the pharmaceutical composition is used for preventing or treating metabolic liver disease in a subject. For example, any animal such as monkeys, dogs, cats, rabbits, marmots, rats, mice, cows, sheep, pigs, goats, *etc.,* birds, fish, *etc.* may be used a subject.

As used herein, the term "administration" refers to the introduction of a prescribed substance to a patient by any suitable method, and the administration route of the composition may be any general route as long as a drug reaches a target tissue. The administration may be intraperitoneal administration, intravenous administration, intramuscular administration, subcutaneous administration, intradermal administration, oral administration, topical administration, intranasal administration, intrapulmonary administration, rectal administration, *etc.,* but is not limited thereto. However, for oral administration, since peptides are digested, it would be preferable to formulate a composition for oral administration to be coated with an active agent, or to be protected from degradation in the stomach. In addition, a pharmaceutical composition may be administered by any device which enables an active substance to travel to a target cell.

The pharmaceutical composition of the present invention may include a pharmaceutically effective amount of a chemical compound, or an isomer or salt thereof. As used herein, the term "pharmaceutically effective amount" refers to an amount sufficient for the treatment of a disease at a reasonable benefit/risk ratio applicable to a medical treatment. In general, for adults (60 kg), an amount of 0.001 mg/day to 1,000 mg/day, specifically, an amount of 0.01 mg/day to 100 mg/day, more specifically, an amount of 0.05 mg/day to 10 mg/day, and even more specifically, an amount of 0.05 mg/day to 2 mg/day may be administered once or several times per day.

In a specific embodiment of the present invention, a NASH mouse was orally administered with a vehicle supplemented with trametinib at a dose of 0.2 mg/kg (in a volume of 10 mL/kg) once per day from 6 weeks to 9 weeks of age. This dose may be converted into 1 mg/day for a 60 kg adult by applying the formula of "mouse dose (/kg) = human dose (/kg) X 12.3" according to FDA guidelines.

For the object of the present invention, it is preferable that a specific therapeutically effective amount may be differently applied to a certain patient depending on the type and degree of a response to be achieved, a specific composition including whether other formulations are used in some cases, a patient's age, body weight, general health condition, sex, diet, administration time, administration routes, secretion rate of a composition, duration of treatment, various factors including a drug(s) to be used or simultaneously used in combination with the specific composition, and similar factors well known in the medical field.

The therapeutically effective amount may be determined by one of ordinary skill in the art through a conventional dose determination test in the medical field with a set of symptoms provided.

The frequency of administration of the pharmaceutical composition of the present invention is not particularly limited, but may be once per day or several times in divided doses. The pharmaceutical composition of the present invention may be administered as an individual therapeutic agent, or in combination with other therapeutic agent(s). The pharmaceutical composition of the present invention may be administered in combination with a conventional therapeutic agent(s) sequentially or simultaneously. In addition, the pharmaceutical composition may be administered in a single dose or multiple doses. It is important to administer an amount to obtain the maximum effect with a minimum amount without adverse effects considering all of the factors described above, and these factors can be easily determined by one of ordinary skill in the art.

For the prevention or treatment of metabolic liver disease, the pharmaceutical composition of the present invention may be used alone or in combination with an operation, hormone therapy, drug therapy, and a method of using a biological response modifier. For the object of the present invention, the pharmaceutical composition according to the present invention may be administered in combination with one or more additional drugs which are conventionally used in the treatment of metabolic liver disease.

As an example, the composition may be administered in combination with a therapeutic agent for non-alcoholic fatty liver disease. Also the composition may be administered in combination with a therapeutic agent for non-alcoholic steatohepatitis, but is not limited thereto.

When the pharmaceutical composition according to the present invention is administered for the purpose of treating non-alcoholic fatty liver disease or non-alcoholic steatohepatitis, the pharmaceutical composition may be administered in combination with one or more components and/or drugs effective in the treatment or prevention of non-alcoholic fatty liver disease or non-alcoholic steatohepatitis.

Specifically, the trametinib of the present invention or pharmaceutically acceptable salt thereof in combination with the one or more additional drugs may be administered simultaneously, sequentially, or in reverse order. The combination administration may be simultaneous administration at a suitable effective amount, or may be simultaneous, sequential, or reverse-order administration after storage in separate containers.

Examples of the components and/or drugs effective in the treatment or prevention of non-alcoholic fatty liver disease or non-alcoholic steatohepatitis may include thiazolidinediones (TZDs), vitamin E, metformin, statins, ursodeoxycholic acid (UDCA), polyunsaturated fatty acids such as omega 3, *etc.,* angiotensin receptor blockers, pentoxifylline, glucagon-like peptide 1 (GLP-1) receptor agonists, dipeptidyl peptidases 4 (DDP-4) inhibitors, sodium/glucose cotransporter 2 (SGLT2) inhibitors, obeticholic acid (OCA), elafinbranor, telmisartan, *etc.,* but the examples thereof are not particularly limited thereto. Any components and/or drugs known in the art may be used without limitation as long as they are effective in the treatment or prevention of non-alcoholic fatty liver disease or non-alcoholic steatohepatitis.

Still another aspect of the present invention provides a food composition for use in preventing or ameliorating metabolic liver disease, comprising trametinib or a sitologically acceptable salt thereof.

The terms "trametinib", "metabolic liver disease", and "prevention" are described as above.

As used herein, the term "amelioration" refers to any activity that improves or benefits the symptoms of a subject suspected of having or suffering from a subject disease by using a composition comprising trametinib or sitologically acceptable salt thereof.

The food composition according to the present invention may include a formulation such as pills, powders, granules, infusions, tablets, capsules, or liquids, *etc.* Foods to which the composition of the present invention can be added may include, for example, various kinds of foods such as beverages, gums, teas, vitamin complexes, health supplement food, *etc.*

Ingredients of the food composition of the present invention may include other ingredients without limitation in addition to trametinib, which is an essential ingredient, and may contain various herbal medicine extracts, food supplement additives, or natural carbohydrates *etc.* as additional ingredients, like common foods.

In addition, the food supplement additive may include a common food supplement additive used in this field, for example, flavoring agents, aromatics, coloring agents, fillers, stabilizers, *etc.*

Examples of the natural carbohydrate may include common saccharides such as monosaccharides (*e*.*g*., glucose, fructose, etc.); disaccharides (*e*.*g*., maltose, sucrose, etc.); and polysaccharides (*e*.*g*., dextrin, cyclodextrin, *etc.),* and sugar alcohols such as xylitol, sorbitol, erythritol, *etc.* In addition to the above, as flavoring agents, natural flavoring agents (*e*.*g*., rebaudioside A, glycyrrhizine, *etc.)* and synthetic flavoring agents (saccharin, aspartame, *etc.)* may be effectively used.

In addition, the food composition of the present invention may contain various nutritional supplements, vitamins, minerals (electrolytes), aromatics including synthetic aromatics, natural aromatics, *etc.,* coloring agents, and fillers (cheese, chocolate, *etc.),* pectic acid and salts thereof, alginic acid and salts thereof, organic acids protective colloidal thickeners, pH adjusting agents, stabilizers, preservatives, glycerin, alcohols, carbonating agents used in carbonated drinks, *etc.* In addition, the food composition of the present invention may contain natural fruit juices and fruit flesh for the preparation of fruit juices and vegetable drinks. These ingredients may be used independently or in combination.

As used herein, the health supplement food includes health functional food, health food, *etc.* The health functional food is synonymous with a food for special health use (FoSHU), and refers to a food which has a high medical or medicinal effects and which has been processed so that biological regulation functions are efficiently exerted in addition to nutritional supply. In particular, "functional" refers to regulating nutrients with respect to structures and functions of a human body or obtaining beneficial effects, such as physiological actions, *etc.,* which are useful for health use. The food of the present invention can be prepared by a method of commonly used in the art. For the preparation of the food, raw materials and ingredients commonly added in the art may be added to the food. In addition, the formulation of the food may be prepared without limitation as long as the formulation is acceptable as a food. The food composition of the present invention may be prepared in various types of formulations. Unlike general drugs, the food composition uses a food as an ingredient, and thus has an advantage in that there are no side effects, *etc.* which may be caused by long-term intake of a drug, and has excellent portability.

Still another aspect of the present invention provides a feed composition for use in preventing or ameliorating metabolic liver disease, comprising trametinib or a sitologically acceptable salt thereof.

The terms "trametinib", "metabolic liver disease", "prevention", and "amelioration" are as described above.

As used herein, the term "feed" refers to a substance that supplies organic or inorganic nutrients required for maintaining animal life. The feed may include nutrients for energy, such as proteins, lipids, vitamins, minerals, *etc.* that are required for animals including livestock, *etc.,* and may be a plant-based feed such as grains, nuts, processed food by-products, seaweed, fiber, fats and oils, starches, meals, grain by-products, *etc.,* or an animal-based feed such as proteins, inorganic matter, fats and oils, minerals, single-cell proteins, *etc.,* but the feed is not limited thereto.

The feed of the present invention may include a powder feed, a solid feed, a moist pellet feed, a dry pellet feed, an extruder pellet (EP) feed, a raw feed, *etc.,* but is not limited thereto.

The feed composition of the present invention may include binders, emulsifiers, preservatives, *etc.* which can be added to prevent quality deterioration. The feed composition may include a feed additive. The feed additive may include amino acids, vitamins, enzymes, flavoring agents, non-protein nitrogen compounds, silicates, buffers, extracts, oligosaccharides, *etc.* which are added to a feed to increase utility. In addition, the feed additive may further include a feed mixture(s), *etc.,* but is not limited thereto.

### Mode for the Invention

Hereinafter, the present invention will be described in detail through exemplary embodiments below. However, these exemplary embodiments are for illustrative purposes only.

### Example 1. Establishment of Non-Alcoholic Steatohepatitis (NASH) Model Mice

Pathogen-free C57BL/6 mice on the 14^{th} day of pregnancy were obtained from Japan SLC, Inc. (Japan).

NASH was established in male mice by a single subcutaneous injection of 200 µg streptozotocin (STZ, Sigma, USA) 2 days after birth and feeding with a high-fat diet (CLEA Japan Inc., Japan) *ad libitum* after 4 weeks of age (day 28 ± 2).

### Example 2. Administration of Compounds to NASH Model Mice

On the day before the start of administration based on the body weight of mice, the NASH mice at 6 weeks of age (day 42±2) prepared in Example 1 were randomly divided into 3 groups of 8 mice.

The present inventors selected trametinib as a substance expected to have a therapeutic effect on non-alcoholic steatohepatitis through a screening method using artificial intelligence (AI) deep learning technology. In order to identify whether the substance has an effect on non-alcoholic steatohepatitis, the 3 groups of mice were each intraperitoneally or orally administered with the following compounds at an amount of about 100 µL/mouse. As the negative control group of trametinib in the present invention, the vehicle administration group was treated with a solvent of the same amount used for compound dissolution. Telmisartan (Boehringer Ingelheim GmbH, Germany) was used in the positive control group. The telmisartan is a compound which has anti-NASH, anti-fibrosis, and anti-inflammatory effects in NASH and in a liver disease animal model related to NASH.
- Group 1 (vehicle administration group: negative control group): 8 NASH mice were intraperitoneally administered with a vehicle (4% DMSO in saline) in a volume of 5 mL/kg once daily from 6 weeks to 9 weeks of age.
- Group 2 (telmisartan administration group: positive control group): 8 NASH mice were orally administered with purified water supplemented with telmisartan at a dose of 10 mg/kg once daily from 6 weeks to 9 weeks of age.
- Group 3 (trametinib administration group: experimental group): 8 NASH mice were intraperitoneally administered with a vehicle supplemented with trametinib at a dose of 2 mg/kg once daily from 6 weeks to 9 weeks of age.

The body weight of each mouse was measured daily during the administration period, and the survival rate, clinical symptoms, and behaviors of each mouse were monitored daily.

### Example 3. Histological Analysis of Liver Tissue

For histological analysis of liver tissue, mice in Groups 1 to 3 were sacrificed at 9 weeks of age, and the liver was excised therefrom.

### 3-1. Hematoxylin and Eosin (H&E) Staining

For H&E staining, after sectioning liver tissue, a liver tissue slide fixed with paraffin was immersed in a xylene solution for 3 minutes to remove the paraffin. Thereafter, in order to remove the xylene, the liver tissue slide was immersed in 100% ethanol for 3 minutes and then hydrated with distilled water. Subsequently, the liver tissue slide was immersed in a hematoxylin solution for 5 to 10 minutes and then hydrated with distilled water for 5 minutes, followed by quickly immersing it twice in a 1% HCl solution, and then hydrating with distilled water. Then, the liver tissue slide was immersed in a 1% ammonia solution for 2 minutes to stain the tissue blue. Thereafter, the liver tissue stained blue was immersed in an eosin solution for 2 minutes, followed by immersing the resultant in ethanol for about 3 to 5 minutes. Lastly, the liver tissue was immersed in a xylene solution, whereupon the thus-stained liver fragment was observed under a microscope.

The photomicrographs of the liver section stained with H&E, which were observed under a microscope, are as shown in FIG. 1.

As illustrated in FIG. 1, the liver section of the vehicle administration group exhibited serious fat accumulation, inflammatory cell infiltration, and ballooning, which is a phenomenon in which hepatic cells swell. However, it was found that the above pathological conditions of the liver tissue of the trametinib administration group were improved compared to those of the vehicle administration group, and that the trametinib administration group exhibited an excellent effect compared to the telmisartan administration group (*i*.*e*., positive control group). In particular, it was shown that the ballooning of hepatic cells was remarkably reduced in the liver tissue of the trametinib administration group.

### 3-2. Estimation of NAFLD Activity Score

The NAFLD activity score (NAS) is a combined value obtained by considering each condition of steatosis, inflammation, and ballooning, which are observed in a liver section stained with H&E. The NAFLD score was calculated according to Kleiner's criteria (Kleiner DE *et al.,* 2005). The composition of an NAFLD activity score is shown in Table 1 below.

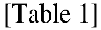

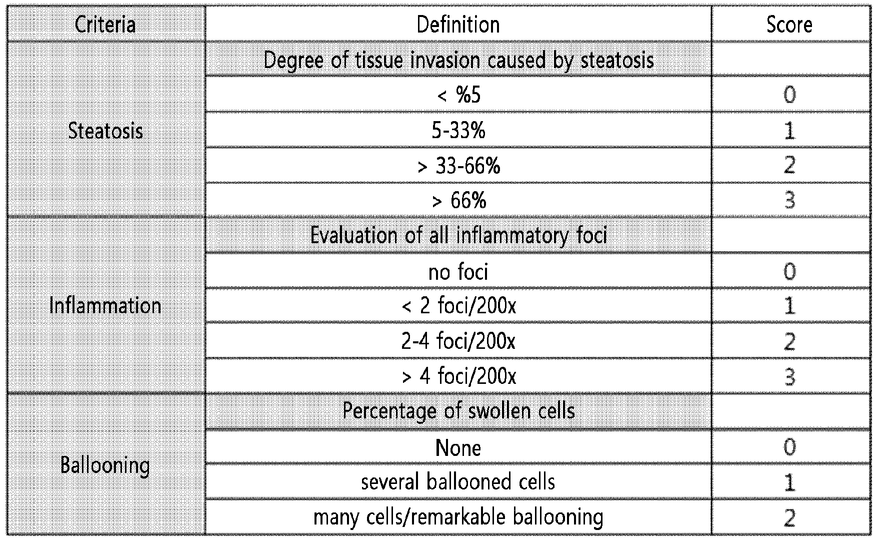

Steatosis, inflammation, and ballooning scores were determined according to severity of symptoms based on observation in H&E-stained liver sections.

Specifically, as a result of comparing steatosis scores, as shown in FIG. 2, it was found that there were 6 subjects with a steatosis score of 1 in the vehicle administration group, whereas 1 subject in the telmisartan administration group had a steatosis score of 1, and all of the subjects in the trametinib administration group had a steatosis score of 0. This showed that the trametinib administration group has a superior effect of reducing steatosis as compared to the telmisartan administration group *(i.e.,* positive control group). Based on the results above, it was confirmed that trametinib showed an excellent effect of reducing steatosis.

As a result of comparing ballooning scores, as shown in FIG. 4, there were 6 subjects with a ballooning score of 1 in the vehicle administration group, and 1 subject (No. 5) with a ballooning score of 2 in the vehicle administration group as well. However, all of the subjects in the trametinib administration group had a ballooning score of 0. Based on the results above, it was found that trametinib remarkably reduced the ballooning of hepatic cells in the liver tissue of the non-alcoholic fatty liver disease animal model.

The NAFLD activity score, in which the steatosis score, inflammatory score, and ballooning score above are comprehensively considered, is shown in FIG. 5.

Specifically, it was found that the NAFLD activity score of the vehicle administration group was 4.38±0.52, while that of the telmisartan administration group was 2.75±0.46 (*p*<0.001 relative to the vehicle administration group), and that the trametinib administration group had an NAFLD activity score of 2.63±0.52 (p<0.001 relative to the vehicle administration group). That is, the trametinib administration group showed a significant decrease in the NAFLD activity score as compared to the vehicle administration group (*p*<0.001), and it was similar or superior to the telmisartan administration group *(i.e.,* positive control group).

Based on the results above, by comprehensively considering the scores of steatosis, inflammation, and ballooning in liver tissue, it was confirmed that trametinib had an effect of treating non-alcoholic fatty liver disease.

### 3-3. Sirius Red Staining

Sirius red staining is a staining method most frequently used in diagnosis to evaluate the level of tissue destruction, which enables observation of the fibrosis level of inflammation induced by non-alcoholic steatohepatitis.

For sirius red staining, a sirius red reagent was maintained in an equilibrium state and stirred gently. After removing paraffin from a liver tissue section fixed with paraffin, the liver tissue section was hydrated with distilled water and then completely immersed in a sirius red solution for 60 minutes. The liver tissue slide was quickly rinsed twice with an acetic acid solution, followed by rinsing the slide with 100% ethanol. After removing the slide, the liver tissue was mounted with a synthetic resin and then observed under a microscope.

As a result, as shown in FIG. 6, it was found that the fibrosis of the mice of the trametinib administration group was alleviated as compared to that of the vehicle administration group, and was even further alleviated as compared to that of the positive control group.

### 3-4. Estimation of Percentage of Fibrosis Area

As a result of estimating a percentage of a fibrosis area due to inflammation induced by non-alcoholic steatohepatitis through the sirius red staining performed in Example 3-3, it was found that the trametinib administration group exhibited a remarkable decrease in the fibrosis area as compared to the vehicle administration group (*p* <0.0001), and it was similar to the telmisartan administration group *(i.e.,* positive control group) (FIG. 7).

In addition, a value of a sirius red-positive area was determined according to severity of fibrosis development based on observation in sirius red-stained liver sections. As a result of identifying values of a sirius red-positive area through the sirius red staining performed in Example 3-3, it was found that the fibrosis area score of the vehicle administration group was 0.89±0.19, whereas that of the telmisartan administration group *(i.e.,* positive control group) was 0.40±0.15 (*p*<0.0001 relative to the vehicle administration group), and similarly, that of the trametinib administration group was 0.50±0.13 (*p*<0.001 relative to the vehicle administration group) (FIG. 7).

Based on the results above, it was confirmed that trametinib exhibits a significant decrease in the fibrosis area due to inflammation induced by non-alcoholic steatohepatitis, and thus has an effect for treating non-alcoholic fatty liver disease.

### Example 4. Measurement of Liver Weight and Liver-to-Body Weight Ratio

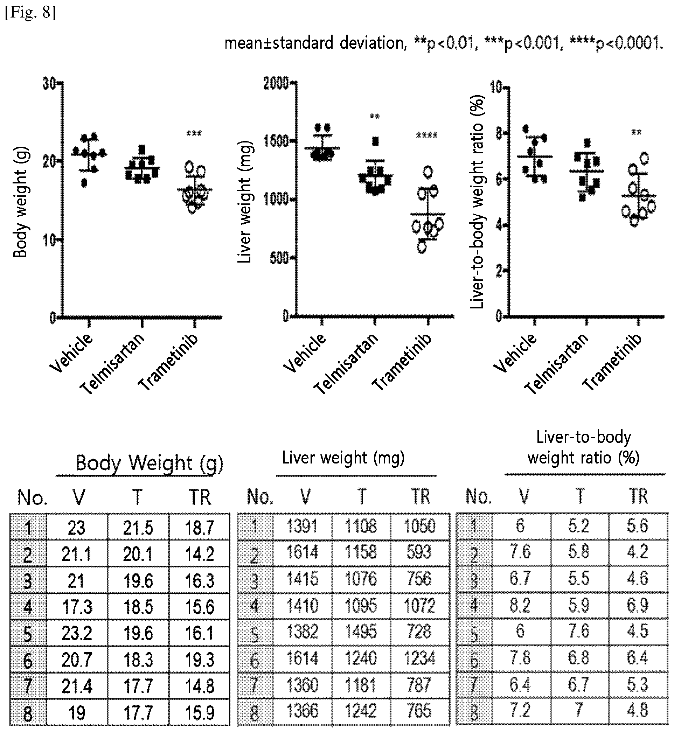

The body weight, liver weight, and liver-to-body weight ratio of mice from each group according to Example 2 are shown in FIG. 8.

As illustrated in FIG. 8, the average liver weight (mg) of the trametinib administration group (873±218 mg) was remarkably reduced by 40% as compared to that of the vehicle administration group (1444±106 mg). The liver-to-body weight ratio (%) of the trametinib administration group (5.3±0.96%) was reduced as compared to that of the vehicle administration group (7.0±0.8%).

Based on the results above, it was found that trametinib can reduce the liver weight that has increased due to non-alcoholic fatty liver disease.

### Example 5. Oral Administration of Trametinib to NASH Model Mice

In order to determine a safe oral administration dose of trametinib, as in Example 2, the NASH mice in Example 1 were randomly divided into 3 groups of 7 or 8 mice. Thereafter, the mice of 3 groups were orally administered with the following compounds at about 100 µL/mouse.
- Group 1 (vehicle administration group: negative control group): 7 NASH mice were orally administered with a vehicle (1% DMSO in saline) in a volume of 10 mL/kg once daily from 6 weeks to 9 weeks of age.
- Group 2 (telmisartan administration group: positive control group): 8 NASH mice were orally administered with purified water supplemented with telmisartan at a dose of 10 mg/kg (in a volume of 10 mL/kg) once daily from 6 weeks to 9 weeks of age.
- Group 3 (trametinib administration group: experimental group): 8 NASH mice were orally administered with a vehicle supplemented with trametinib at a dose of 0.2 mg/kg (in a volume of 10 mL/kg) once daily from 6 weeks to 9 weeks of age.

The body weight of each mouse was measured daily during the administration period, and the survival rate, clinical symptoms, and behaviors of each mouse were monitored daily.

### Example 6. Histological Analysis of Liver Tissue

For histological analysis of liver tissue, mice in Groups 1 to 3 of Example 5 were sacrificed at 9 weeks of age, and then H&E staining, NAFLD activity score measurement, and sirius red staining were performed according to the method of Example 3.

### 6-1. H&E Staining

As a result of H&E staining of liver tissue according to the method of Example 3-1, as shown in FIG. 9, it was found that the pathological conditions of the liver tissue of the mice administered with trametinib were improved as compared to those of the vehicle administration group (*i*.*e*., negative control group), and that the efficacy of the trametinib administration group was more superior to that of the telmisartan administration group *(i.e.,* positive control group).

### 6-2. Estimation of NAFLD Activity Score

The NAFLD activity score for liver tissue was estimated according to the method of Example 3-2.

Specifically, as a result of comparing steatosis scores, as shown in FIG. 10, there were 6 subjects with a steatosis score of 1 in the vehicle administration group, while there were 3 subjects with a steatosis score of 1 in the trametinib administration group. In addition, the steatosis score (mean ± standard deviation) of the trametinib administration group was 0.38±0.52, which was significantly reduced as compared to that of the vehicle administration group (0.86±0.38). Based on the results above, it was confirmed that trametinib had an effect of reducing steatosis.

As a result of comparing inflammation scores, as shown in FIG. 11, it was found that the inflammation score of the trametinib administration group (1.75±0.71) was lower than that of the vehicle administration group (2.71±0.49), and thus, trametinib had an effect of reducing inflammation.

The NAFLD activity score, in which the steatosis score, inflammatory score, and ballooning score above are comprehensively considered, is shown in FIG. 13.

Specifically, it was found that the NAFLD activity score of the vehicle administration group was 4.29±0.49, while that of the telmisartan administration group was 2.25±0.46 and that of the trametinib administration group was 2.88±1.13. That is, the trametinib administration group showed a significant decrease in the NAFLD activity score as compared to the vehicle administration group.

Based on the results above, by comprehensively considering the scores of steatosis, inflammation, and ballooning in liver tissue, it was confirmed that trametinib had an effect of treating non-alcoholic fatty liver disease.

### 6-3. Sirius Red Staining

As a result of sirius red staining of liver tissue according to the method of Example 3-3, as shown in FIG. 14, it was found that the pathological conditions of the trametinib administration group were improved as compared to those of the vehicle administration group, and that the effect of the trametinib administration group on fibrosis occurrence was even more superior or similar to that of the telmisartan administration group (*i.e*., positive control group).

### 6-4. Estimation of Percentage of Fibrosis Area

According to the method of Example 3-4, a percentage of a fibrosis area due to inflammation and a value of a sirius red-positive area were each estimated through sirius red staining.

As a result, as shown in FIG. 15, the percentage of the fibrosis area of the trametinib administration group was reduced as compared to that of the vehicle administration group.

In addition, as a result of identifying a value of a sirius red-positive area, it was found that the fibrosis area score of the vehicle administration group was 0.69±0.13, while that of the telmisartan administration group *(i.e.,* positive control group) was 0.44±0.06, and that of the trametinib administration group was 0.34±0.15, which is lower than that of the telmisartan administration group (FIG. 15).

Based on the results above, it was confirmed that trametinib exhibited a significant reduction in the fibrosis area due to inflammation induced by non-alcoholic steatohepatitis, and thus had an effect for treating non-alcoholic fatty liver disease.

### Example 7. Measurement of Liver-to-Body Weight Ratio

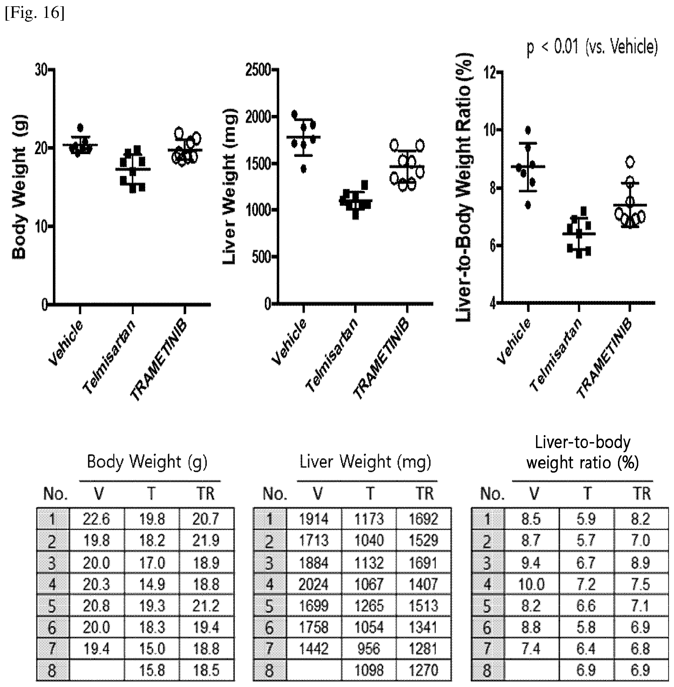

The body weight, liver weight, and liver-to-body weight ratio of the 9-week-old mouse of Example 5 are as shown in FIG. 16.

As illustrated in FIG. 16, the average liver weight (mg) of the trametinib administration group (1466±169 mg) was reduced as compared to that of the vehicle administration group (1776±189 mg). The liver-to-body weight ratio (%) of the trametinib administration group (7.4±0.8%) was reduced as compared to that of the vehicle administration group (8.7±0.8%).

Based on the results above, it was found that trametinib can reduce the liver weight that has increased due to non-alcoholic fatty liver disease.

### Example 8. Statistical Analysis

Statistical analysis of the data of the Examples above was performed using the Bonferroni Multiple Comparison Test. *p* <0.05 was regarded as statistically significant.

As confirmed in the results of the Examples above, the composition of the present invention, which comprises trametinib, reduces steatosis, inflammation, or ballooning in liver tissue where non-alcoholic fatty liver has been induced; reduces the level of fibrosis of inflammation induced by non-alcoholic steatohepatitis; and reduces the weight of a liver that has increased due to non-alcoholic fatty liver disease. Accordingly, the composition of the present invention can be effectively used for the prevention or treatment of metabolic liver disease including non-alcoholic fatty liver disease, specifically, non-alcoholic steatohepatitis.

In the present invention, detailed disclosure of contents which can be sufficiently recognized or inferred by one of ordinary skill in the art has been omitted. In addition to the exemplary embodiments described above, various modifications are possible within the scope of not changing the technical spirit or essential features of the present invention. Accordingly, the present invention can be implemented in other ways different from those that are specifically explained and illustrated in the present specification, and this can be appreciated by one of ordinary skill in the art.

## Claims

1. A pharmaceutical composition for use in preventing or treating non-alcoholic fatty liver disease (NAFLD), comprising trametinib or a pharmaceutically acceptable salt thereof.

2. The pharmaceutical composition for use in preventing or treating NAFLD according to claim 1, wherein the trametinib is represented by Chemical Formula I below:

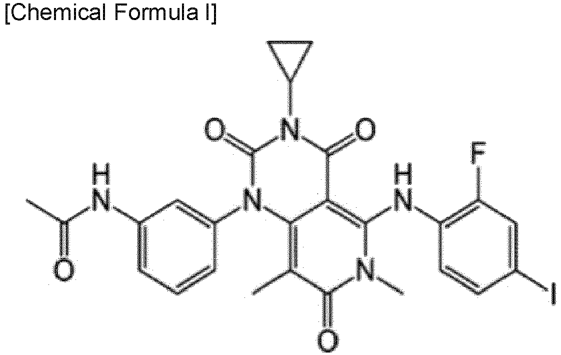

3. The pharmaceutical composition for use in preventing or treating NAFLD according to claim 1, wherein the composition reduces steatosis, inflammation, or ballooning.

4. The pharmaceutical composition for use in preventing or treating NAFLD according to claim 1, wherein the composition reduces fibrosis or cirrhosis.

5. The pharmaceutical composition for use in preventing or treating NAFLD according to claim 1, wherein the composition further comprises a pharmaceutically acceptable carrier, excipient, or diluent.

6. The pharmaceutical composition for use in preventing or treating NAFLD according to claim 1, wherein the composition is administered in combination with a therapeutic agent for non-alcoholic fatty liver disease (NAFLD).

7. The pharmaceutical composition for use in preventing or treating NAFLD according to claim 1, wherein the composition is administered in combination with a therapeutic agent for non-alcoholic steatohepatitis (NASH).

8. The pharmaceutical composition for use in preventing or treating NAFLD according to claim 6, wherein the therapeutic agent for non-alcoholic fatty liver disease (NAFLD) is one or more selected from the group consisting of thiazolidinediones (TZDs), vitamin E, metformin, statins, ursodeoxycholic acid (UDCA), polyunsaturated fatty acids, angiotensin receptor blockers, pentoxifylline, glucagon-like peptide 1 (GLP-1) receptor agonists, dipeptidyl peptidase 4 (DPP-4) inhibitors, sodium/glucose co-transporter 2 (SGLT2) inhibitors, obeticholic acid (OCA), elafibranor, and telmisartan.

9. The pharmaceutical composition for use in preventing or treating NAFLD according to claim 1, wherein the non-alcoholic fatty liver disease is one or more selected from the group consisting of non-alcoholic steatohepatitis (NASH), non-alcoholic fatty liver (NAFL), NAFLD-associated liver fibrosis and hepatocirrhosis.

10. A food composition for use in preventing or ameliorating non-alcoholic fatty liver disease, comprising trametinib or a sitologically acceptable salt thereof.

11. The food composition for the use of claim 10, wherein the trametinib is represented by Chemical Formula I below:

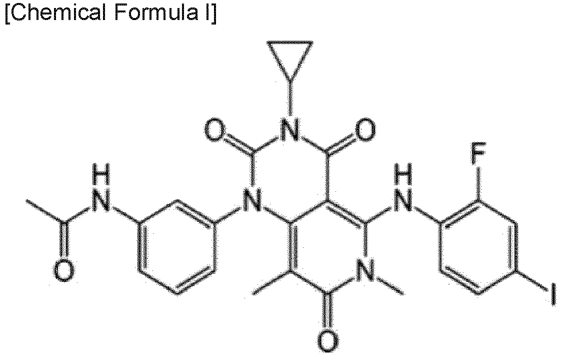

## Patentansprüche

1. Pharmazeutische Zusammensetzung zur Verwendung beim Vorbeugen oder Behandeln nicht alkoholischer Fettlebererkrankung (NAFLD), umfassend Trametinib oder ein pharmazeutisch unbedenkliches Salz davon.

2. Pharmazeutische Zusammensetzung zur Verwendung beim Vorbeugen oder Behandeln von NAFLD nach Anspruch 1, wobei Trametinib durch die folgende chemische Formel I dargestellt ist:

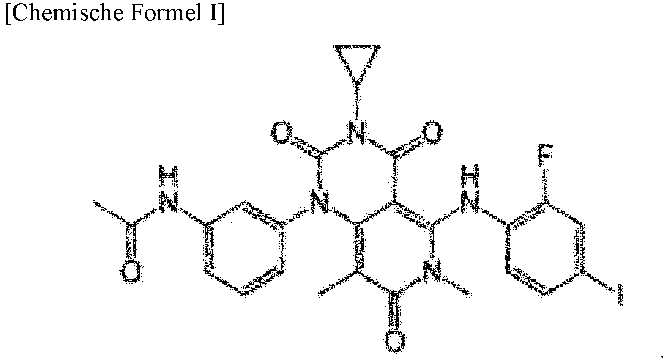

3. Pharmazeutische Zusammensetzung zur Verwendung beim Vorbeugen oder Behandeln von NAFLD nach Anspruch 1, wobei die Zusammensetzung Steatose, Entzündung oder Ballonierung reduziert.

4. Pharmazeutische Zusammensetzung zur Verwendung beim Vorbeugen oder Behandeln von NAFLD nach Anspruch 1, wobei die Zusammensetzung Fibrose oder Zirrhose reduziert.

5. Pharmazeutische Zusammensetzung zur Verwendung beim Vorbeugen oder Behandeln von NAFLD nach Anspruch 1, wobei die Zusammensetzung ferner ein pharmazeutisch unbedenkliches Trägermittel, Hilfsmittel oder Verdünnungsmittel umfasst.

6. Pharmazeutische Zusammensetzung zur Verwendung beim Vorbeugen oder Behandeln von NAFLD nach Anspruch 1, wobei die Zusammensetzung in Kombination mit einem therapeutischen Mittel gegen nicht alkoholische Fettlebererkrankung (NAFLD) verabreicht wird.

7. Pharmazeutische Zusammensetzung zur Verwendung beim Vorbeugen oder Behandeln von NAFLD nach Anspruch 1, wobei die Zusammensetzung in Kombination mit einem therapeutischen Mittel gegen nicht alkoholische Steatohepatitis (NASH) verabreicht wird.

8. Pharmazeutische Zusammensetzung zur Verwendung beim Vorbeugen oder Behandeln von NAFLD nach Anspruch 6, wobei das therapeutische Mittel gegen nicht alkoholische Fettlebererkrankung (NAFLD) eines oder mehrere ist, das/die aus der Gruppe ausgewählt ist/sind, die aus Thiazolidindionen (TZD), Vitamin E, Metformin, Statinen, Ursodesoxycholsäure (UDCA), mehrfach ungesättigten Fettsäuren, Angiotensin-Rezeptorblockern, Pentoxifyllin, Glucagon-like-Peptide-1(GLP-1)-Rezeptoragonisten, Dipeptidylpeptidase-4(DPP-4)-Inhibitoren, Natrium/Glucose-Cotransporter-2(SGLT2)-Inhibitoren, Obeticholsäure (OCA), Elafibranor und Telmisartan besteht.

9. Pharmazeutische Zusammensetzung zur Verwendung beim Vorbeugen oder Behandeln von NAFLD nach Anspruch 1, wobei die nicht alkoholische Fettlebererkrankung eine oder mehrere ist, die aus der Gruppe ausgewählt ist/sind, die aus nicht alkoholischer Steatohepatitis (NASH), nicht alkoholischer Fettleber (NAFL), NAFLD-assoziierter Leberfibrose und Hepatozirrhose besteht.

10. Lebensmittelzusammensetzung zur Verwendung beim Vorbeugen oder Lindern einer nicht alkoholischen Fettlebererkrankung, umfassend Trametinib oder ein diätologisch unbedenkliches Salz davon.

11. Lebensmittelzusammensetzung zur Verwendung nach Anspruch 10, wobei das Trametinib durch die folgende chemische Formel I dargestellt ist:

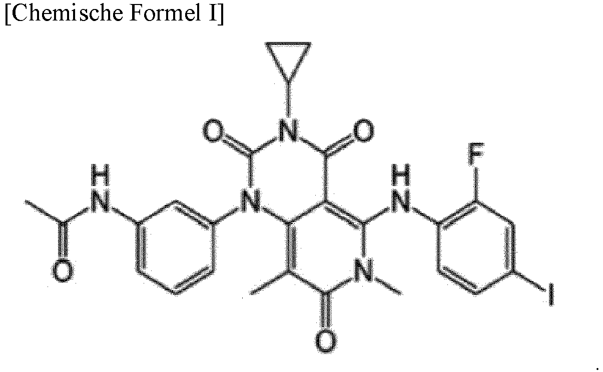

## Revendications

1. Composition pharmaceutique pour utilisation dans la prévention ou le traitement de la maladie du foie stéatosique non alcoolique (NAFLD), comprenant du trametinib ou un sel pharmaceutiquement acceptable de celui-ci.

2. Composition pharmaceutique pour utilisation dans la prévention ou le traitement de la NAFLD selon la revendication 1, où le trametinib est représenté par la formule chimique I ci-dessous :

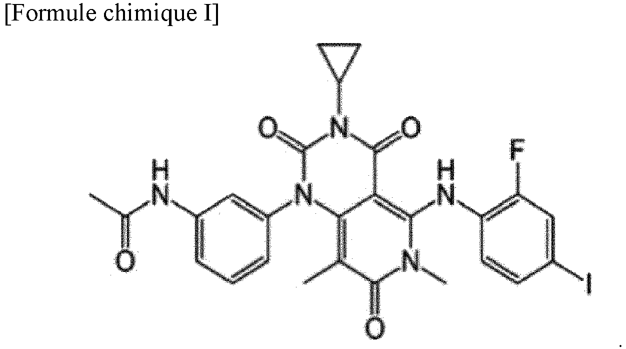

3. Composition pharmaceutique pour utilisation dans la prévention ou le traitement de la NAFLD selon la revendication 1, où la composition réduit la stéatose, l'inflammation ou les ballonnements.

4. Composition pharmaceutique pour utilisation dans la prévention ou le traitement de la NAFLD selon la revendication 1, où la composition réduit la fibrose ou la cirrhose.

5. Composition pharmaceutique pour utilisation dans la prévention ou le traitement de la NAFLD selon la revendication 1, où la composition comprend en outre un véhicule, excipient ou diluant pharmaceutiquement acceptable.

6. Composition pharmaceutique pour utilisation dans la prévention ou le traitement de la NAFLD selon la revendication 1, où la composition est administrée conjointement avec un agent thérapeutique pour la maladie du foie stéatosique non alcoolique (NAFLD).

7. Composition pharmaceutique pour utilisation dans la prévention ou le traitement de la NAFLD selon la revendication 1, où la composition est administrée conjointement avec un agent thérapeutique pour stéatohépatite non alcoolique (NASH).

8. Composition pharmaceutique pour utilisation dans la prévention ou le traitement de la NAFLD selon la revendication 6, où l'agent thérapeutique pour la maladie du foie stéatosique non alcoolique (NAFLD) est un ou plusieurs agents sélectionnés dans le groupe constitué de : thiazolidinédiones (TZDs), vitamine E, metformine, statines, acide ursodéoxycholique (UDCA), acides gras polyinsaturés, bloqueurs des récepteurs de l'angiotensine, pentoxifylline, agonistes des récepteurs de peptides type glucagon 1 (GLP-1), inhibiteurs de la dipeptidylpeptidase 4 (DPP-4), inhibiteurs des co-transporteurs sodium/glucose 2 (SGLT2), acide obéticholique (OCA), elafibranor et telmisartan.

9. Composition pharmaceutique pour utilisation dans la prévention ou le traitement de la NAFLD selon la revendication 1, où la maladie du foie stéatosique non alcoolique est une ou plusieurs des maladies sélectionnées dans le groupe constitué de : stéatohépatite non alcoolique (NASH), stéatose hépatique non alcoolique (NAFL), fibrose hépatique et hépatocirrhose liées à la NAFLD.

10. Composition alimentaire pour utilisation dans la prévention ou l'amélioration de la maladie du foie stéatosique non alcoolique, comprenant le trametinib ou un sel sitologiquement acceptable de celui-ci.

11. Composition alimentaire pour utilisation selon la revendication 10, où le trametinib est représenté par la formule chimique I ci-dessous :
